(19) Europäisches Patentamt
European Patent Office
Office européen des brevets

(11) EP 3 434 367 A1

(12) **EUROPEAN PATENT APPLICATION**
published in accordance with Art. 153(4) EPC

(43) Date of publication:
**30.01.2019 Bulletin 2019/05**

(21) Application number: **17769728.1**

(22) Date of filing: **15.02.2017**

(51) Int Cl.:
***B01J 23/89*** *(2006.01)* ***C07D 213/73*** *(2006.01)*

(86) International application number:
**PCT/JP2017/005584**

(87) International publication number:
**WO 2017/163681 (28.09.2017 Gazette 2017/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.03.2016 JP 2016058062**

(71) Applicant: **N.E. Chemcat Corporation**
**Tokyo 105-6124 (JP)**

(72) Inventors:
- **SUZUKA, Hiroyasu**
  **Bando-shi**
  **Ibaraki 306-0608 (JP)**
- **MIZUSAKI, Tomoteru**
  **Bando-shi**
  **Ibaraki 306-0608 (JP)**
- **NAKAYA, Yusuke**
  **Bando-shi**
  **Ibaraki 306-0608 (JP)**
- **WADA, Yoshiyuki**
  **Bando-shi**
  **Ibaraki 306-0608 (JP)**

(74) Representative: **Mewburn Ellis LLP**
**City Tower**
**40 Basinghall Street**
**London EC2V 5DE (GB)**

# (54) REACTION COMPOSITION AND REACTION SYSTEM USING THIS

(57) An aromatic nitro compound has a structure in which a nitro group and a halogen atom, in a separated state, are directly bonded as substituents to the ring structure of the same ring; a reaction composition is provided which, in a hydrogenation reaction of the nitro group of said aromatic nitro compound, allows selectively hydrogenating the nitro group, and sufficiently reducing the separation of the halogen atom from the ring; also provided is a reaction system that uses this reaction composition. This reaction composition includes a solvent, and a catalyst which, with the aforementioned aromatic nitro compound as reactant, is used in a hydrogenation reaction of at least one of the one or more nitro groups of said reactant. The catalyst includes a carrier, and Fe oxide particles and Pt particles supported by the carrier. Further, the components of the solvent satisfy expression (1): $0.00\% \leqq \{100\times(Vh/Vs)\} \leqq 10.00\%$. In expression (1), Vh represents the volume (L) of water introduced other than reaction-produced water, and Vs represents the volume (L) of the organic solvent which can dissolve at least part of the aforementioned reactant.

EP 3 434 367 A1

## Description

TECHNICAL FIELD

The present invention relates to a reaction composition which includes a catalyst and an organic solvent, and a reaction system used the reaction composition. More specifically, the present invention relates to a reaction composition which includes a catalyst used in a hydrogenation reaction of one or more nitro groups of an unsaturated cyclic compound (aromatic nitro compound) having a structure where one or more nitro groups are directly bonded the ring skeleton, and an organic solvent. Further, the present invention relates to a reaction system which realizes, by using the reaction composition of the present invention, that the nitro group of the aromatic nitro compound of a reactant can be selectively hydrogenated, and the removal of the halogen atoms from the ring can be sufficiently reduced.

BACKGROUND ARTS

An aromatic halogen amine having a structure where one or more nitro groups and one or more amino groups are directly bonded as substituents to a ring skeleton of the same ring while separated from each other, is an important raw material of a medicine, a dye, an insecticide, a herbicide.

The aromatic halogen amine can be prepared, for example, by the hydrogenation reaction (catalytic hydrogenation reaction) of an aromatic halogen nitro compound having the corresponding chemical structure (aromatic nitro compound having a structure where one or more nitro groups and one or more halogen atoms are directly bonded as substituents to a ring skeleton of the same ring while separated from each other).

In this hydrogenation reaction, in addition to the reduction reaction (desired hydrogenation reaction) where the nitro group is reduced to an amino group, a reaction where a halogen atom is removed from the ring and replaced by a hydrogen atom (undesired side reaction. Hereinafter referred to as "dehalogenation reaction" as necessary) also happened. Hydrogen halide produced by the dehalogenation reaction corrodes the reaction vessel.

Therefore, in the hydrogenation reaction of the aromatic halogen nitro compound, it is important to sufficiently reduce the dehalogenation reaction and to selectively reduce the nitro group.

Various proposals have been made for selectively reducing the nitro group of the aromatic halogen nitro compound.

For example, in order to avoid the dehalogenation reaction, Patent Document 1 proposes that the hydrogenation reaction of the aromatic halogen nitro compound (an aromatic nitro derivative having a halogen atom bonded to an aromatic nucleus in Patent Document 1) is carried out in the presence of tungsten carbide and a strong acid (sulfuric acid, phosphoric acid, hydrochloric acid, hydrobromic acid).

Further, in order to selectively proceed the hydrogenation of a nitro group of the aromatic halogen nitro compound, Patent Document 2 proposes a noble metal catalyst in which Pt and Cu are finely dispersed on an activated carbon as a carrier in a predetermined supporting amount.

Furthermore, in order to selectively proceed the hydrogenation of a nitro group of the aromatic halogen nitro compound (for example, 1-nitro-3,4-dichlorobenzene, 4-nitrochlorobenzene), Patent Document 3 proposes a catalyst containing a Pt-supporting carbon and Fe oxide-supporting carbon (or Fe hydroxide supporting carbon).

Furthermore, in the hydrogenation reaction of a nitro group of the aromatic halogen nitro compound (4-chloro-2,5-dimethoxy-1-nitrobenzene), in order to isolate the reaction product 4-chloro-2,5- dimethoxyaniline without crystallization from the solvent (xylene), Patent Document 4 proposes a reaction system which includes a sulfurized Pt-supporting carbon catalyst, an aliphatic open chain amine (particularly morpholine), an alkaline aqueous solution {compound which gives a pH of 8 to 10 in an aqueous solution (for example, disodium borate, sodium formate, sodium acetate, sodium carbonate, disodium hydrogen phosphate, sodium hydroxide) }, a predetermined amount of water (30 mL in Example 1 of Patent Document 4 , 1.5 mL of water contained in the catalyst), and a predetermined amount of an aromatic organic solvent (675 mL in Example 1 of Patent Document 4)

Incidentally, the present applicant submits, as publications where the above-mentioned publicly-known inventions are described, the following publications:

PRIOR ART LITERATURE

Patent Document

Patent Document 1: Japanese Patent Laid-Open Application H5-271105
Patent Document 2: Japanese Patent Laid-Open Application H6-71178

Patent Document 3: United State Patent No.4212824
Patent Document 4: Japanese Patent Laid-Open Application H2-45452

SUMMARY OF INVENTION

Problem to be Solved by the Invention

However, in the hydrogenation reaction of the aromatic halogen nitro compound, from the viewpoint of sufficiently reducing the dehalogenation reaction and selectively reducing the nitro group, the present inventors have found that there is still room for improvement even in the abovementioned conventional technique.
The present invention has been completed considering the abovementioned technical circumstances, and can provide a reaction composition which, in a nitro group hydrogenation reaction of an aromatic nitro compound having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.

Means for solving the problems

As the results of the present inventors' intensive study for solving the above problems, the present inventors have found that a structure of a reaction composition where a catalyst in which, in addition to Pt particles, Fe oxide particles are supported on a carrier is mixed with a predetermined organic solvent is effective, and then the present invention has been completed.
More specifically, the present invention is configured by the following technical elements.
Namely, the present invention provides:

(N1) a reaction composition comprising
a catalyst which is used in a hydrogenation reaction of at least one among one or more nitro groups present in a reactant to an amino group, the reactant being an aromatic nitro compound having a structure in which one or more nitro groups and one or more halogen atoms are directly bonded as substituents to a ring skeleton of the same ring while separated from each other, and
an organic solvent,

wherein:

the catalyst comprises a support, and Pt particles and Fe oxide particles supported on the support, and
the constituent element of the organic solvent satisfies the following condition of the equation (1):

$$0.00\% \leq \{100 \text{ x } (Vh/Vs)\} \leq 10.00\% \qquad (1)$$

in the equation (1), Vh being a volume (L) of an introduced water other than the yielded water, and Vs being a volume (L) of the organic solvent which can dissolve at least a part of the reactant.

In carrying out the selective hydrogenation reaction of the nitro group of the aromatic halogen nitro compound, the present inventors have found that, in a chemical system of a catalyst in which Pt particles and Fe oxide particles coexist, when the content of water is set to a relatively small amount as mentioned above, though the detailed mechanism has not been found, in the hydrogenation reaction of the nitro group of the aromatic halogen nitro compound, the reaction composition satisfying the above configuration can selectively hydrogenate the nitro group, and the removal of the halogen atom from the ring can be sufficiently reduced.
Here, the reaction composition of the present invention described in (N1) may further contain
(N2) the aromatic nitro compound.
Further, in this case, the reaction composition described in (N2) may further contain
(N3) an aromatic amine obtained by the hydrogenation reaction of the aromatic nitro compound as a reaction product and having at least one amino group.
Furthermore, from the viewpoint of obtaining the effect of the present invention more reliably, in the reaction composition of the present invention described in any one of (N1) to (N3), it is preferable that
(N4) the constituent elements of the solvent satisfy the condition of the following equation (2):

$$1.00\% \leq \{100 \text{ x } (Vh/Vs)\} \leq 5.00\% \qquad (2)$$

Further, any one of the reaction compositions of any one of (N1) to (N4) may further contain (N5) a dehydrating agent.
When previously mixing the dehydrating agent, the water content in the reaction composition of the present invention can be reduced, and there is a case where the effects of the present invention can be obtained easily.
Further, the present invention provides a reaction system comprising a reaction vessel which can accommodate at least one of the reaction compositions according to any one of (N1) to (N6) as a reactant.
According to the reaction system of the present invention, since the reaction composition of the present invention is used, it can be realized that the nitro group of the aromatic nitro compound of a reactant can be selectively hydrogenated, and the removal of the halogen atoms from the ring can be sufficiently reduced.

Effect of the invention

The present invention can provide the reaction composition which, in a nitro group hydrogenation reaction of an aromatic nitro compound (aromatic halogen nitro compound) having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.
Furthermore, the present invention can provide the reaction system which can realize that the nitro group of the aromatic nitro compound of a reactant can be selectively hydrogenated, and the removal of the halogen atoms from the ring can be sufficiently reduced.

Means for Solving the Problem

In the following, preferred embodiments of the present invention are explained in detail.
The reaction composition of the present embodiment contains the catalyst and the solvent.
The catalyst contains the support, and the Pt particles and the Fe oxide particles supported on the support. Further, the organic solvent has chemical properties that the solvent can dissolve at least a part of the reactants.
The catalyst is not particularly limited as long as the catalyst contains the support, and the Pt particles and the Fe oxide particles supported on the support.
The support of the catalyst is not particularly limited as long as the support can support the Pt particles and the Fe oxide particles and has a large surface area. Examples include a carbon-based material such as an activated carbon, a pulverized activated carbon, a grassy carbon (GC), a fine carbon, a carbon black, graphite, or a carbon fiber, or a glass-based or a ceramic-based material such as an oxide, and may be properly used.
The specific surface area of the support is preferably 500 $m^3/g$ or more, more preferably 800 $m^3/g$ or more, and still more preferably 1000 $m^3/g$ or more.
In the Fe oxide particles of the catalyst contained in the reaction composition, the Fe oxide is not particularly limited, but preferably contains $Fe_2O_3$ as a main component.
In the reaction composition of the present embodiment, the constituent element of the solvent further satisfies the conditions of the following equation (1):

$$0.00\% \leq \{100 \text{ x } (Vh/Vs)\} \leq 10.00\% \qquad (1)$$

in the equation (1), Vh being a volume (L) of an introduced water other than the yielded water, and Vs being a volume (L) of the organic solvent which can dissolve at least a part of the reactant.
Furthermore, in this case, it is more preferable that the constituent element of the solvent further satisfies the condition of the following equation (2):

$$1.00\% \leq \{100 \text{ x } (Vh/Vs)\} \leq 5.00\% \qquad (2)$$

Further, the reaction composition may further contain the dehydrating agent.
The dehydrating agent is not particularly limited as long as, in the state of being mixed with the Pt particles and the Fe oxide particles, the agent has stability such that the chemical reaction does not proceed with these substances at normal temperature and normal pressure. From the viewpoint of availability and the like, preferable examples of the

dehydrating agent include zeolite, sodium sulfate, magnesium sulfate.

The organic solvent contained in the reaction composition is not particularly limited as long as the solvent has chemical properties capable of dissolving at least a part of the reactant (aromatic halogen nitro compound). Preferred examples include toluene, xylene, benzene, chlorobenzene, dichlorobenzene, and an alcohol having 1 to 3 carbon atoms. A mixture thereof at an optional ratio may be used.

The reactant (aromatic halogen nitro compound) in the reaction system where the reaction composition is used is not particularly limited, and it is preferable that the reactant has the structure represented by the following general formula (C1):

[Chemical Formula 1]

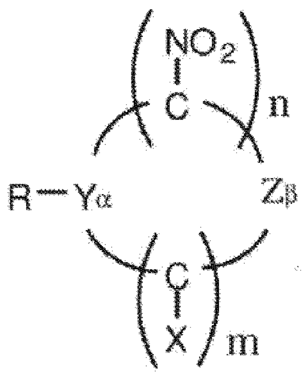

· · · (C1)

In the formula (C1),

n represents an integer of 1 or more,
m represents an integer of 1 or more,
$\alpha$ represents an integer of 0 or more,
$\beta$ represents an integer of 0 or more, and

$$5 \leq (n + m + \alpha + \beta) \leq 6,$$

R represents hydrogen atom, amino group, hydroxyl group or a mono-valent or more-valent organic group having at least one carbon atom,
X represents any one of halogen atoms,
Y represents, in the case of $\alpha$ = 1, an atom of C, N, O, or S, and, in the case of $\alpha \geqq 2$, at least two atoms selected from the group consisting of C, N, O and S,
Z represents, in the case of $\beta$ = 1, an atom of C, N, O, or S, and, in the case of $\beta \geq 2$, at least two atoms selected from the group consisting of C, N, O and S, and
Y and Z may be the same or different from each other.

Here, when $\alpha$ is 2 or more, R may be a divalent organic group bonded to two adjacent Y atoms. In this case, the aromatic nitro compound may have a structure of a condensed ring compound.

In addition, R which is an organic group having mono- or more-valence, may have a structure in which the moiety bonding to Ya is represented by "-O-" or "-S-".

For example, in the formula (C1), in the case of $\alpha$ = 2, $Y_1$ = C, $Y_2$ = N, R = H, $\beta$ = 2, $Z_1$ = C, $Z_2$ = C, m = 1, X = Br, n = 1, (n + m + $\alpha$ + $\beta$) = 6, (C1) is 2-bromo-5-nitropyridine represented by the following formula (C1-1).

[Chemical Formula 2]

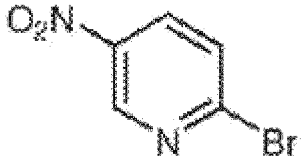 ・・・(C1-1)

The reactant (aromatic halogen nitro compound) in the reaction system where the reaction composition is used may have the structure represented by the following general formula (C2):

[Chemical Formula 3]

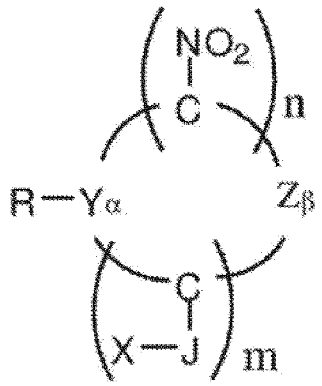 ・・・(C2)

In the formula (C2),

n represents an integer of 1 or more,
m represents an integer of 1 or more,
$\alpha$ represents an integer of 0 or more,
$\beta$ represents an integer of 0 or more, and

$$5 \leq (n + m + \alpha + \beta) \leq 6,$$

R represents hydrogen atom, amino group, hydroxyl group or a mono-valent or more-valent organic group having at least one carbon atom,
X represents any one of halogen atoms,
Y represents, in the case of $\alpha$ = 1, an atom of C, N, O, or S, and, in the case of $\alpha \geqq 2$, at least two atoms selected from the group consisting of C, N, O and S,
Z represents, in the case of $\beta$ = 1, an atom of C, N, O, or S, and, in the case of $\beta \geqq 2$, at least two atoms selected from the group consisting of C, N, O and S,
Y and Z may be the same or different from each other, and
J represents a divalent organic group having one or more carbon atoms, and in the case of $m \geqq 2$, J may be the same or different from each other.

In addition, R which is an organic group having mono- or more-valence, may have a structure in which the moiety bonding to $Y\alpha$ is represented by "-O-" or "-S-".

For example, in the formula (C2), the moiety represented by the "X-J-" may have the following structures represented by the formulae (C2-1), (C2-2), (C2-3), and (C2-4).

X-C-(C=O)-N- (C2-1)

X-C=C-C- (C2-2)

X-C-C- (C2-3)

[Chemical Formula 4]

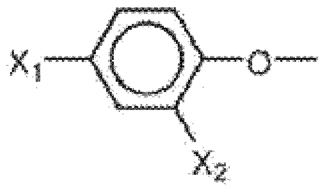

· · · (C2-4)

In the formula (C2-4), $X_1$ and $X_2$ each represent any one of halogen atoms, and $X_1$ and $X_2$ may be the same or different from each other.

In the reaction composition of the present invention, the aromatic nitro compound which is the reactant may be previously contained.

Further, in this case, the reaction composition may further contain an aromatic amine obtained by the hydrogenation reaction of the aromatic nitro compound as a reaction product and having at least one amino group.

The "aromatic amine having one or more amino groups" desirably is the aromatic halogen amine as the main product, but within the range where the effect of the present invention can be obtained (within the range being allowable in the reaction system to be used), a case where an aromatic amine which is a dehalogenated body is slightly contained may be allowable.

<Preparation process of Reaction composition>

The preparation process of the reaction composition is not particularly limited, and any combination of known processes can be employed.

As the catalyst contained in the reaction composition, for example, a catalyst where the Pt particles and the Fe oxide particles are supported on the support may be obtained by subjecting a dispersion containing a Pt compound, a Fe compound and water to reduction treatment to the support . The supporting of Pt and the supporting of Fe oxide may be carried out at the same time as described above, or one may be carried out first and then the other may be later.

When mixing the catalyst and the solvent (organic solvent, and water used as occasion demand), considering chemical properties of each component, the mixing order thereof is not particularly limited as long as these components can sufficiently mix without causing any undesired chemical reaction.

Further, when mixing the catalyst, the solvent and the dehydrating agent, the mixing order thereof is not particularly limited as long as these components can sufficiently mix without causing any undesired chemical reaction.

When containing the reactant previously in the reaction composition, the mixing order is not particularly limited as long as the reactant can be sufficiently mixed without causing any undesired chemical reaction with other constituents.

The reaction composition of the present embodiment may be accommodated so as to be storable in a package such as a container or a bag after preparation. The internal structure of the package in contact with the reaction composition should be configured so as not to cause any chemical reaction with the reaction composition. When accommodating the reaction composition in the reaction composition packaging body, it is possible to easily move the reaction composition from the preparation place to the use place when the production place and the use place of the reaction composition are different.

Next, the preferred embodiments of the reaction system of the present invention are explained.

The reaction system of the present embodiment has a configuration which includes a reaction vessel which can accommodate the reaction composition of the present invention as the reactant.

The reaction system of the present embodiment may have a configuration including a reaction vessel capable of accommodating the reaction composition of the present invention as the reactant, and the other configuration is not particularly limited. As the configuration of the reaction system of the present embodiment, there can be employed the configurations which are the same as those of the known reaction system used for the hydrogenation reaction of at least one among one or more nitro groups present in a reactant to an amino group, the reactant being an aromatic nitro compound (aromatic halogen nitro compound) having a structure in which one or more nitro groups and one or more halogen atoms are directly bonded as substituents to a ring skeleton of the same ring while separated from each other.

In the reaction system of the present embodiment, the reaction composition of the present invention may be previously included as the constituent element. In this case, in the reaction system, the reaction composition of the

present invention may be included in the reaction vessel previously, or may be previously accommodated and maintained in a container which is other than the reaction vessel, and when the reaction is carried out, the reaction composition may be transported from the other container to the reaction vessel.

Furthermore, the reaction system of this embodiment may have the configuration where the system further includes the other container which previously accommodates and maintains the constituent elements of the reaction composition of the present invention, before carrying out the hydrogenation reaction at the place where the hydrogenation reaction is carried out, and the constituent elements of the reaction composition are introduced into a predetermined container to prepare the reaction composition, and them the prepared reaction composition is used for the hydrogenation reaction.

In this case, the reaction system may have the other containers which can previously accommodate and maintain each of the constituent elements of the reaction composition of the present invention, or may have the other container which can previously accommodate and maintain a part of the constituent elements of the reaction composition of the present invention. For example, the reaction system may further have the other container that can previously accommodate and maintain the organic solvent respectively, and the catalyst may be brought into from the other manufacturing place of the catalyst.

According to the reaction system of the present embodiment, since the reaction composition of the present invention is used, it is possible to realize the hydrogenation reaction where the nitro group of the aromatic nitro compound of the reactant can be selectively hydrogenated, and the removal of the halogen atoms from the ring can be sufficiently reduced.

## EXAMPLE

In the following, the present invention is explained in detail by referring working examples, but the present invention is not limited by the following working examples.

### <Preparation of Reaction composition>

(Example 1)

As the catalyst, a catalyst where the Pt particles and the Fe oxide particles were carried on a carbon support {trade name "NE-01M02", content of Pt: 1.0 wt%, content of Fe: 0.20 wt% available from N. E. CHEMCAT Co. (hereinafter referred to as "Pt-FeOx/C" as necessary)} was prepared.

In the Pt-FeOx/C, the carbon support is an activated carbon (specific surface area based on BET measurement is 900: $m^2/g$), and the Fe oxide particles are $Fe_2O_3$ as a main component ($Fe_2O_3$ is approximately 100% based on XPS analysis).

As the organic solvent, a commercially available toluene was prepared.

A reaction composition was obtained by mixing 127.0 mg (water content: 0.141 mL) of Pt-FeOx/C powder, and 10 mL of toluene (organic solvent).

(Example 2)

The reaction composition of Example 2 was prepared in the same preparation conditions and the same raw materials as Example 1 except that 127.0 mg (water content: 0.141 mL) of the same powder of Pt-FeOx/C catalyst as in Example 1 was subjected to drying treatment (using a dryer, holding in air at 300 °C for 15 minutes) to adjust the water content measured with a moisture content meter to 0 mL was used.

(Example 3)

The reaction composition of Example 3 was prepared in the same preparation conditions and the same raw materials as Example 1 except that the reaction composition was prepared by adding the dehydrating agent to 127.0 mg (water content: 0.141 mL) of the same powder of Pt-FeOx/C catalyst as in Example 1, and then adding 10 mL of toluene.

As the dehydrating agent, 2 g of commercially available zeolite having a structure code "LTA" type (Molecular Sieves 4A) was used. The amount of the dehydrating agent is enough to dehydrate all moisture contained in the Pt-FeOx/C catalyst.

(Example 4) to (Example 9)

The reaction compositions of Example 4 to Example 9 were prepared in the same preparation conditions and

the same raw materials as Example 1 except that the value of {100 x (Vh/Vs)} was changed to the value shown in Table 1.

]

(Comparative Example 1)

The reaction composition of Comparative Example 1 was prepared in the same preparation conditions and the same raw materials as in Example 1 except that 1 mL of water was added to the same reaction composition as Example 1.

<Hydrogenation Reaction>

The hydrogenation reaction (hydrogenation reaction of nitro group) represented by the following reaction scheme (C1-11) was achieved by using the reaction compositions of Example 1 to Example 3 and Comparative Example 1.

[Chemical Formula 5]

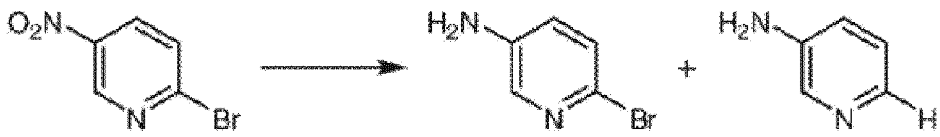

・・・(C1-11)

[Chemical Formula 6]

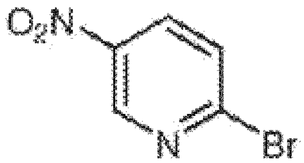

・・・(C1-1)

[Chemical Formula 7]

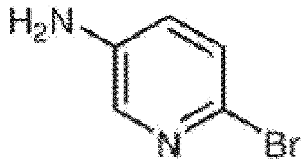

・・・(C1-2)

[Chemical Formula 8]

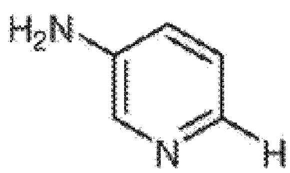

・・・(C1-3)

Here, in the hydrogenation reaction represented by the reaction scheme (C1-11), 2-bromo-5-nitropyridine represented by the formula (C1-1) has a heterocyclic structure having an N atom. Compared to 2-bromo-5-nitrobenzene which has a structure in which the N atom in this heterocyclic ring is replaced with a C atom, 2-bromo-5-nitropyridine is a reactant which promotes not only the main hydrogenation reaction of the nitro group (main product being 5-amino-2-bromopyridine represented by the formula (C1-2)), but also promotes easily the side reaction of the debromination reaction (by-product being 3-aminopyridine represented by the formula (C1-3).

(Reaction Conditions)

Reactant: 2.5 mmol of 2-Bromo-5-nitrobenzene represented by the formula (C1-1)
Pressure of hydrogen: 0.6 MPa
Reaction temperature: 50 °C (Example 1, Example 2, Examples 4 to Example 9), 100 °C (Example 3, Comparative Example 1)
Reaction time: 5 hours

The results obtained for the reaction compositions of Examples 1 to 9 and Comparative Example 1 to Comparative Example 9 are shown in Table 1 and Table 2.

[Table 1]

| | Catalyst structure | Vh Water volume /mL | Vs Org. Solvent (Toluen) volume/mL | 100x (Vh/Vs) % | Conversion % | Selectivity % 5-amino-2-bromopyridine main product | Selectivity % 3-aminopyridine by-product (DeBr body) |
|---|---|---|---|---|---|---|---|
| Ex.1 | Pt-FeOx/C | 0.141 | 10.0 | 1.41 | 100 | 81.5 | 18.5 |
| Ex.2 | Pt-FeOx/C | 0.000 | 10.0 | 0.00 | 100 | 88.5 | 11.5 |
| Ex.3 | Pt-FeOx/C | 0.000 | 10.0 | 0.00 | 100 | 86.0 | 14.0 |
| Ex.4 | Pt-FeOx/C | 0.050 | 10.0 | 0.50 | 100 | 81.9 | 18.1 |
| Ex.5 | Pt-FeOx/C | 0.100 | 10.0 | 1.00 | 100 | 82.9 | 17.1 |
| Ex.6 | Pt-FeOx/C | 0.136 | 10.0 | 1.36 | 100 | 82.0 | 18.0 |
| Ex.7 | Pt-FeOx/C | 0.200 | 10.0 | 2.00 | 100 | 81.0 | 19.0 |
| Ex.8 | Pt-FeOx/C | 0.300 | 10.0 | 3.00 | 100 | 81.8 | 18.2 |
| Ex.9 | Pt-FeOx/C | 0.460 | 10.0 | 4.60 | 100 | 80.3 | 19.7 |
| Com.Ex.1 | Pt-FeOx/C | 1.141 | 10.0 | 11.41 | 100 | 21.5 | 78.5 |
| Com.Ex.2 | Pt/C | 0.113 | 10.0 | 1.13 | 100 | 48.9 | 51.1 |

From the results shown in Table 1, when the reaction compositions of Example 1 to Example 9 which satisfy the constitution of the present invention are compared with the reaction composition of Comparative Example 1, it has been clear that the debromination reaction to 3-aminopyridine represented by the formula (C1-3) was sufficiently reduced, and the selectivity of the desired 5-amino-2-bromopyridine represented by the formula (C1-2) was improved.

From the aforementioned results, it has been clear that, in a nitro group hydrogenation reaction of an aromatic nitro compound having a structure in which nitro groups and halogen atoms are directly bonded as substituents to a ring skeleton of the same ring while separated from each other, the reaction composition of the present examples is capable of selectively hydrogenating the nitro groups and sufficiently reducing the removal of the halogen atoms from the ring.

Industrial Applicability

The reaction composition of the present invention has the catalyst activities that, in a nitro group hydrogenation reaction of an aromatic nitro compound (aromatic halogen nitro compound) having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.

Therefore, the present invention contributes to the development of efficient mass production technology of the aromatic halogen amine which is an important raw material of medicines, dyes, insecticides and herbicides, and further contributes to the development of the industries of pharmaceuticals, dyes, insecticides, herbicides.

## Claims

1. A reaction composition comprising
a catalyst which is used in a hydrogenation reaction of at least one among one or more nitro groups present in a reactant to an amino group, the reactant being an aromatic nitro compound having a structure in which one or more nitro groups and one or more halogen atoms are directly bonded as substituents to a ring skeleton of the same ring while separated from each other, and
an organic solvent,
wherein:

    the catalyst comprises a support, and Pt particles and Fe oxide particles supported on the support, and
    the constituent element of the organic solvent satisfies the following condition of the equation (1):

$$0.00\% \leq \{100 \text{ x } (Vh/Vs)\} \leq 10.00\% \qquad (1)$$

    in the equation (1), Vh being a volume (L) of an introduced water other than the yielded water, and Vs being a volume (L) of the organic solvent which can dissolve at least a part of the reactant.

2. The reaction composition according to claim 1, further comprising the aromatic nitro compound.

3. The reaction composition according to claim 2, further comprising an aromatic amine obtained by the hydrogenation reaction of the aromatic nitro compound as a reaction product and having at least one amino group.

4. The reaction composition according to any one of claims 1 to 3, wherein the constituent element of the organic solvent satisfies the following condition of the equation (2):

$$1.00\% \leq \{100 \text{ x } (Vh/Vs)\} \leq 5.00\% \qquad (2)$$

5. The reaction composition according to any one of claims 1 to 4, further comprising a dehydrating agent.

6. A reaction system comprising a reaction vessel which can accommodate at least one of the reaction compositions according to any one of claims 1 to 5 as a reactant.

## INTERNATIONAL SEARCH REPORT

International application No.
PCT/JP2017/005584

A. CLASSIFICATION OF SUBJECT MATTER
*B01J23/89*(2006.01)i, *C07D213/73*(2006.01)i

According to International Patent Classification (IPC) or to both national classification and IPC

B. FIELDS SEARCHED

Minimum documentation searched (classification system followed by classification symbols)
B01J23/89, C07D213/73

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WANG, Xiangdong, et al., Enhanced Catalytic Hydrogenation Activity and Selectivity of Pt-$M_xO_y/Al_2O_3$ (M = Ni, Fe, Co) Heteroaggregate Catalysts by in Situ Transformation of PtM Alloy Nanoparticles, The journal of physical chemistry C, 2013.03.20, 117, ISSN:1932-7455, p. 7294-7302, particularly, ABSTRACT, EXPERIMENTAL SECTION, Table 2 | 1-4,6<br>5 |
| X<br>A | HAN, Xiaoxiang, et al., Influence of support and transition metal (Cr, Mn, Fe, Co, Ni and Cu)on the hydrogenation of p-chloronitrobenzene over supported platinum catalysts, Catalysis Today, 93-95, ISSN:0920-5861, 2004.07.08, p. 433-437, particularly, Abstract, Experimental, Table 7 | 1-4,6<br>5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 April 2017 (14.04.17) | 09 May 2017 (09.05.17) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

INTERNATIONAL SEARCH REPORT

International application No. PCT/JP2017/005584

C (Continuation). DOCUMENTS CONSIDERED TO BE RELEVANT

| Category* | Citation of document, with indication, where appropriate, of the relevant passages | Relevant to claim No. |
|---|---|---|
| X<br>A | XU, Xiangsheng, et al., A highly active and chemoselective assembled Pt/C(Fe) catalyst for hydrogenation of o-chloronitrobenzene, Applied Catalysis A : General, 2012.04.04, 429-430, ISSN:0926-860X, p. 17-23, particularly, ABSTRACT, Experimental, Table 2 | 1-4,6<br>5 |
| A | JP 6-71178 A (Degussa AG.), 15 March 1994 (15.03.1994), & US 5512529 A & EP 573910 A1 | 1-6 |
| A | JP 2000-302738 A (Wakayama Seika Kogyo Co., Ltd.), 31 October 2000 (31.10.2000), (Family: none) | 1-6 |
| A | US 5068436 A (E. I. DU PONT DE NEMOURS AND CO.), 26 November 1991 (26.11.1991), (Family: none) | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5271105 B
- JP H671178 B
- US 4212824 A
- JP H245452 B